# EUROPÄISCHE PATENTSCHRIFT

(11) **EP 0 465 458 B1**
(45) Veröffentlichungstag und Bekanntmachung des Hinweises auf die Patenterteilung: **04.01.1995**
(21) Anmeldenummer: 91890127.3
(22) Anmeldetag: 19.06.1991
(51) Int. Cl.: A61M 1/00, A61M 5/158, A61B 5/14, A61M 25/00

(54) **Zweilumige Entnahmenadel für Körperflüssigkeiten**
Dual lumen needle for withdrawal of body fluids
Aiguille à double lumière pour retirer les liquides du corps

(30) Priorität: 04.07.1990 AT 1425/90
(43) Veröffentlichungstag der Anmeldung: 08.01.1992
(60) Teilanmeldung: 94107954.3
(73) Patentinhaber: AVL Medical Instruments AG, 8207 Schaffhausen (CH)
(72) Erfinder: Skrabal, Falko, Prof. Dr., A-8010 Graz (AT); Kleinhappl, Erich, Ing., A-8062 Weinitzen (AT)
(74) Vertreter: Krause, Walter, Dr. Dipl.-Ing.

(56) Entgegenhaltungen:
- EP-A- 0 000 041
- EP-A- 0 107 810
- EP-A- 0 191 599
- EP-A- 0 243 341
- CH-A- 662 739
- US-A- 4 403 983
- US-A- 4 772 268

## Beschreibung

Die Erfindung betrifft eine zweilumige Entnahmenadel für Körperflüssigkeiten, beispielsweise Blut oder Gewebeflüssigkeit, mit im wesentlichen parallel geführten ersten und zweiten elastischen Kanülen, wobei die erste Kanüle eine nach dem Einstechen der Entnahmenadel entfernbare Einführnadel aufweist, deren geschliffenes Ende über die erste Kanüle hinausragt, wobei ein Griffteil der Entnahmenadel neben Anschlüssen für die beiden Kanülen eine durch einen elastischen Stopfen verschlossene separate Öffnung zur Entnahme der Einführnadel aufweist.

In der Medizin gibt es eine Reihe von Anwendungsgebieten für zweilumige Nadeln, die immer dann von Vorteil sind, wenn durch ein Lumen dem Körper eine Substanz zugeführt und durch das andere Lumen diese Substanz, ein Reaktionsprodukt oder eine Körperflüssigkeit entnommen werden soll.

Beispielsweise wird bei der Gewebeperfusion dem Gewebe über ein Lumen einer zweilumigen Nadel eine Perfusionsflüssigkeit zugeführt, welche nach einer kurzen Equilibrierungsphase über das andere Lumen der Nadel wieder gewonnen wird. Die Außenwand der Nadel weist dabei Öffnungen auf, um die Kontaktfläche mit dem Gewebe zu vergrößern.

So sind beispielsweise aus der WO 88/05643 mehrere Ausführungsvarianten von zweilumigen Nadeln bekanntgeworden, welche im wesentlichen aus zwei ineinanderliegenden Stahlröhrchen bestehen. Doppellumige Stahlnadeln können mit relativ geringem Gesamtdurchmesser hergestellt werden, wodurch die Schmerzbelastung des Patienten minimiert ist und weisen zudem aufgrund des verwendeten Materials die für das Einstechen in das Gewebe nötige Festigkeit auf. Nachteilig ist lediglich die Steifigkeit der Nadel, welche den Tragekomfort bei längerer Liegezeit verringert.

Aus der WO 88/05643 ist weiters ein Katheter bzw. eine Entnahmenadel aus elastischem Material bekannt, deren inneres Lumen im Querschnitt sternförmig abstehende Septen aufweist, wobei deren kapillare Zwischenräume das vom anliegenden Gewebe begrenzte äußere Lumen der Nadel bilden. Der biegsame Katheter befindet sich beim Einstechen in das Gewebe in einer Hohlnadel, welche nach dem Einstechen wieder zurückgezogen wird, sodaß nur der elastische Katheter im Gewebe verbleibt. Nachteilig bei dieser Ausführungsform ist der durch die Hohlnadel vergrößerte Gesamtdurchmesser, welcher die Schmerzbelastung des Patienten erhöht, sowie der vergrößerte Manipulationsaufwand.

Aus der EP-A 0 191 599 ist eine zweilumige Entnahmenadel bekanntgeworden, welche sich allerdings nur für Dialysezwecke eignet. Die Öffnungen der beiden Lumen sind axial möglichst weit voneinander entfernt, um zu verhindern, daß von der Dialysevorrichtung in ein Blutgefäß eingebrachtes Blut sofort wieder vom zweiten Lumen abgesaugt wird. Weiters ist ein gewisser Manipulationsaufwand erforderlich, um nach dem Entfernen der Einführnadel aus einer der beiden Kanülen einen Anschluß zur Dialysevorrichtung herzustellen, ohne mit Blut in Kontakt zu kommen.

Aus der EP-A-0 000 041 ist eine zweilumige Entnahmenadel für Köperflüssigkeiten der eingangs genannten Art bekannt, mit im wesentlichen parallel geführten ersten und zweiten elastischen Kanülen, wobei die erste Kanüle eine nach dem Einstechen der Entnahmenadel entfernbare Einführnadel aufweist, deren geschliffenes Ende über die erste Kanüle hinausragt. Der Griffteil der Entnahmenadel weist neben Ahschlüssen für die beiden Kanülen eine durch einen elastischen Stopfen verschlossene separate Öffnung zur Entnahme der Einführnadel auf.

Aufgabe der Erfindung ist es, eine Entnahmenadel der eingangs genannten Art vorzuschlagen, welche einen geringen Gesamtdurchmesser mit einem höheren Tragekomfort bei längerer Liegezeit vereint und auch zur Gewebeperfusion verwendet werden kann. Weiters soll der Manipulationsaufwand vermindert und das Austreten von Körperflüssigkeiten verhindert werden.

Diese Aufgabe wird erfindungsgemäß dadurch gelöst, daß die zweite Kanüle im distalen Endbereich der Entnahmenadel zumindest eine direkte Verbindungsöffnung in die erste Kanüle aufweist, sowie daß die zweite Kanüle zwischen der Einführnadel und der Wand der ersten Kanüle angeordnet ist. Dadurch kann bereits innerhalb der Entnahmenadel für eine Strömungsumkehr, beispielsweise einer Perfusionsflüssigkeit, gesorgt werden. Da im Griffteil der Entnahmenadel neben den Anschlüssen für die beiden Kanülen eine separate, durch einen elastischen Stopfen verschlossene Öffnung zur Entnahme der Einführnadel vorgesehen ist, kann auch keine Körperflüssigkeit austreten. Die für das Einstechen der Nadel benötigte Festigkeit wird durch die Einführnadel, beispielsweise aus Stahl, realisiert, wobei der Gesamtdurchmesser durch den Wegfall einer äußeren Hohlnadel klein bleibt. Nach dem Herausziehen der Einführnadel entsteht eine biegsame, elastische, zweilumige Entnahmenadel mit hohem Tragekomfort. Als zweite oder innere Kanüle könnte auch eine Metallkanüle oder eine Hartplastikkanüle verwendet werden, welche aufgrund ihres geringen Durchmessers biegsam ist und zudem mit dem Körper nicht in Kontakt kommt.

In einer ersten, äußerst bedienungsfreundlichen Ausführungsvariante der Erfindung ist vorgesehen, daß der Griffteil einen mit der ersten Kanüle in Verbindung stehenden Hohlraum aufweist, welcher durch den von der Einführnadel durchsetzten elastischen Stopfen verschlossen ist, daß der Griffteil eine Anformung mit den Anschlüssen für die beiden Kanülen aufweist, wobei einer der Anschlüsse in die erste Kanüle und in den Hohlraum im Griffteil mündet und der andere Anschluß mit der zweiten Kanüle verbunden ist.

Eine weitere Ausführungsvariante sieht vor, daß der Hohlraum im Griffteil über einen stufenlosen Konus an den Innendurchmesser der ersten Kanüle angepaßt ist. Das Einführen der zweiten, inneren Kanüle bei der Herstellung der Nadel wird durch den konischen Übergang erleichtert.

Eine weitere vorteilhafte Ausführungsvariante der Erfindung sieht vor, daß sich das Lumen der zweiten Kanüle erst nach dem Entfernen der Einführnadel in das Lumen der ersten Kanüle entfaltet. So lange sich die Einführnadel in der Entnahmenadel befindet, kann das Lumen der zweiten Kanüle zu einem schmalen Schlitz verformt sein, welcher sich nach dem Entfernen der Einführnadel, bedingt durch die unterschiedlichen Druckverhältnisse in den beiden Kanülen, entsprechend entfaltet. Es sind natürlich auch doppellumige Entnahmenadeln mit im wesentlichen unverformbaren Lumen denkbar.

Weiters kann es erfindungsgemäß von Vorteil sein, wenn die Anformung eine Bohrung aufweist, welche in den Hohlraum mündet, wobei in die Bohrung ein Verbindungsstück gasdicht einsetzbar ist, welches die Anschlüsse oder zu den beiden Kanülen führende Leitungen aufweist.

Ein weiteres Merkmal der Erfindung besteht darin, daß das Verbindungsstück mit den beiden Anschlüssen mit einem Schraubteil an der Anformung des Griffstückes befestigt ist. Es bietet sich dafür vor allem ein sogenannter Luer-Lock-Verschluß an.

Die erfindungsgemäße Entnahmenadel eignet sich hervorragend für Anwendungen im Gewebe, wenn die Wand der ersten Kanüle erfindungsgemäß Wandöffnungen aufweist.

Schließlich ist es für spezielle Anwendungen der Erfindung von Vorteil, wenn die erste Kanüle eine Wandöffnung mit einer für bestimmte Bestandteile der Gewebeflüssigkeit selektiv durchlässige Membran aufweist.

Zur sicheren Handhabung der zweilumigen Entnahmenadel wird erfindungsgemäß vorgeschlagen, daß beide Anschlüsse verwechslungssicher in einen Kupplungsteil mit zu- und abführenden Leitungen einführbar sind.

Die Erfindung wird im folgenden anhand von Zeichnungen näher erläutert. Es zeigen:
- Fig. 1: eine zweilumige Entnahmenadel nach der Erfindung und
- Fig 2: ein Detail einer weiteren Ausführungsvariante.

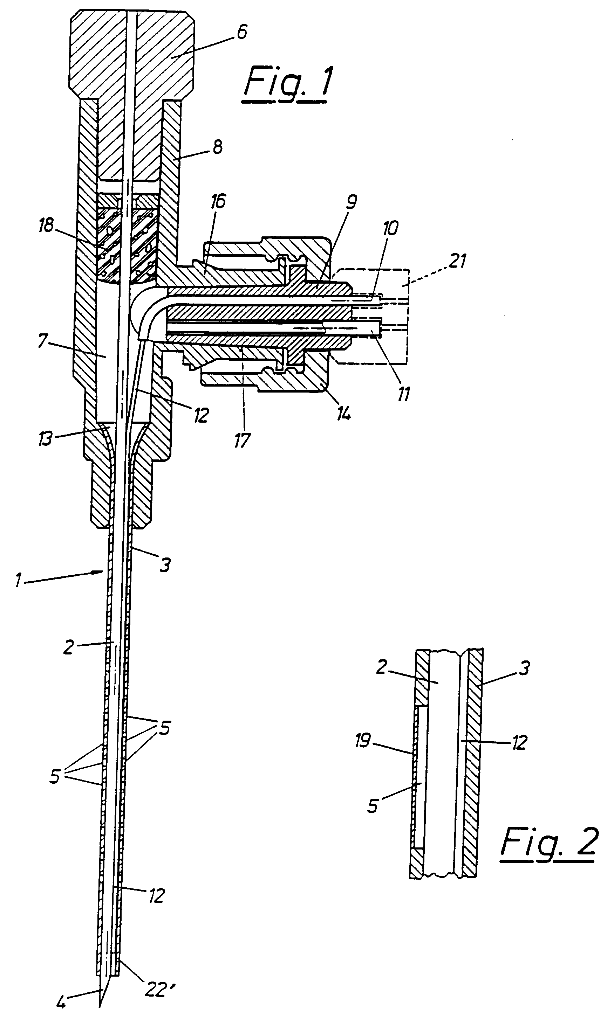

Die in Fig. 1 dargestellte Entnahmenadel 1 weist eine Einführnadel 2, z.B. eine Stahlnadel oder einen Metallstift auf, welcher von einer biegsamen ersten Kanüle 3, z.B. aus Kunststoff, eng umschlossen ist, sodaß nur das geschliffene bzw. spitz oder schneidenförmig ausgeführte Ende 4 der Einführnadel 2 frei bleibt. Die Kanüle 3 weist mehrere, über den Umfang verteilte Wandöffnungen 5 auf, über welche die Perfusionsflüssigkeit mit dem Gewebe in Kontakt kommt. Die Einführnadel 2 ist auf der vom geschliffenen Ende 4 abgewandten Seite mit einer Halterung 6 versehen, welche einen Hohlraum 7 im Griffteil 8 der Nadel 1 verschließt. Für die Anwendung in Blutgefäßen werden Entnahmenadeln 1 verwendet, deren äußere Kanüle 3 keine Öffnungen aufweist. Im Bereich des Endes 4 der Einführnadel 2 kann die Kanüle 3 konisch an den Durchmesser der Einführnadel 2 angepaßt sein, um einen stufenlosen Übergang zu gewährleisten.

Die zweite, innere Kanüle 12 ist zwischen der Einführnadel 2 und der ersten, äußeren Kanüle 3 angeordnet. Durch einen stufenlosen Konus 13 zwischen Hohlraum 7 und äußerer Kanüle 3 wird bei der Herstellung der Nadel das Einsetzen der inneren Kanüle 12 erleichtert. Dabei kann die innere Kanüle 12 deformiert und an die innere Wand der Außenkanüle angedrückt sein und ihr Lumen erst nach dem Herausziehen der Einführnadel 2 entfalten, oder auch in einer Aussparung der Innenwand der äußeren Kanüle 3 liegen. Der Griffteil 8 weist hier eine seitliche Anformung 16 auf, welche ein Verbindungsstück 9 für Anschlüsse 10 und 11 in einer mit dem Hohlraum 7 verbundenen Bohrung 17 aufnimmt. Der Anschluß 10 ist direkt mit der zweiten bzw. inneren Kanüle 12 verbunden, welche bis knapp vor das Ende, eventuell bis zum Ende der ersten bzw. äußeren Kanüle 3 reicht. Die Verbindungsöffnung zwischen zweiter und erster Kanüle ist mit 22' bezeichnet. Als innere Kanüle könnte auch eine dünne Metallkanüle oder eine Kunststoffkanüle mit einem Stahlmandrin verwendet werden. Der zweite Anschluß 11 mündet in den Hohlraum 7 im Griffteil 8 und steht dadurch mit dem Lumen zwischen innerer und äußerer Kanüle in Verbindung. Das Verbindungsstück 9 wird nach dem Einsetzen in die seitliche Anformung 16 durch einen Schraubteil 14, vorzugsweise einer Luer-Lock-Verschraubung, fixiert.

Es ist jedoch auch möglich, die Anschlüsse 10 und 11 direkt in die Anformung 16 einzugießen bzw. einzukleben.

Zwischen der Einmündung der Bohrung 17 in den Hohlraum 7 und der Halterung 6 der Einführnadel 2 ist ein elastischer Stopfen 18 angeordnet, welcher von der Einführnadel 2 durchsetzt wird. Nach dem Einstechen in das Gewebe wird die Einführnadel 2 an ihrer Halterung 6 herausgezogen, wonach sich die Öffnung im elastischen Stopfen 18 schließt und dieser den Hohlraum 7 automatisch abschließt. Die Entnahmenadel 1 ist dann sofort funktionsbereit.

Entsprechend einem in Fig. 2 dargestellten Detail einer Ausführungsvariante der Entnahmenadel 1 kann auch nur eine Öffnung 5 (bzw. eine geringere Anzahl von Öffnungen 5) in der äußeren Kanüle 3 vorgesehen sein, welche eine für bestimmte Probenbestandteile selektive Membran 19 aufweist.

## Patentansprüche

1. Zweilumige Entnahmenadel (1) für Körperflüssigkeiten, beispielsweise Blut oder Gewebeflüssigkeit, mit im wesentlichen parallel geführten ersten und zweiten elastischen Kanülen (3, 12), wobei die erste Kanüle (3) eine nach dem Einstechen der Entnahmenadel (1) entfernbare Einführnadel (2) aufweist, deren geschliffenes Ende (4) über die erste Kanüle (3) hinausragt, wobei ein Griffteil (8) der Entnahmenadel (1) neben Anschlüssen (10, 11) für die beiden Kanülen (3, 12) eine durch einen elastischen Stopfen (18) verschlossene separate Öffnung zur Entnahme der Einführnadel (2) aufweist, **dadurch gekennzeichnet**, daß die zweite Kanüle (12) im distalen Endbereich der Entnahmenadel (1) zumindest eine direkte Verbindungsöffnung (22') in die erste Kanüle (3) aufweist, sowie daß die zweite Kanüle (12) zwischen der Einführnadel (2) und der Wand der ersten Kanüle (3) angeordnet ist.

2. Entnahmenadel nach Anspruch 1, **dadurch gekennzeichnet**, daß der Griffteil (8) einen mit der ersten Kanüle (3) in Verbindung stehenden Hohlraum (7) aufweist, welcher durch den von der Einführnadel (2) durchsetzten elastischen Stopfen (18) verschlossen ist, daß der Griffteil (8) eine Anformung (16) mit den Anschlüssen (10, 11) für die beiden Kanülen (3, 12) aufweist, wobei einer der Anschlüsse (11) in die erste Kanüle (3) und in den Hohlraum (7) im Griffteil (8) mündet und der andere Anschluß (10) mit der zweiten Kanüle (12) verbunden ist.

3. Entnahmenadel nach Anspruch 2, **dadurch gekennzeichnet**, daß der Hohlraum (7) im Griffteil (8) über einen stufenlosen Konus (13) an den Innendurchmesser der ersten Kanüle (3) angepaßt ist.

4. Entnahmenadel nach einem der Ansprüche 1 bis 3, **dadurch gekennzeichnet**, daß sich das Lumen der zweiten Kanüle (12) erst nach dem Entfernen der Einführnadel (2) in das Lumen der ersten Kanüle (3) entfaltet.

5. Entnahmenadel nach einem der Ansprüche 2 bis 4, **dadurch gekennzeichnet**, daß die Anformung (16) eine Bohrung (17) aufweist, welche in den Hohlraum (7) mündet, wobei in die Bohrung (17) ein Verbindungsstück (9) gasdicht einsetzbar ist, welches die Anschlüsse (10, 11) oder zu den beiden Kanülen (3, 12) führende Leitungen aufweist.

6. Entnahmenadel nach Anspruch 5, **dadurch gekennzeichnet**, daß das Verbindungsstück (9) mit den beiden Anschlüssen (10, 11) mit einem Schraubteil (14) an der Anformung (16) des Griffstückes befestigt ist.

7. Entnahmenadel nach einem der Ansprüche 1 bis 6, **dadurch gekennzeichnet**, daß die Wand der ersten Kanüle Wandöffnungen (5) aufweist.

8. Entnahmenadel nach einem der Ansprüche 1 bis 6, **dadurch gekennzeichnet**, daß die erste Kanüle (3) eine Wandöffnung (5) mit einer für bestimmte Bestandteile der Gewebeflüssigkeit selektiv durchlässigen Membran (19) aufweist.

9. Entnahmenadel nach einem der Ansprüche 1 bis 8, **dadurch gekennzeichnet**, daß beide Anschlüsse (10, 11) der Entnahmenadel (1) verwechslungssicher in einen Kupplungsteil (21) mit zu- und abführenden Leitungen einführbar sind.

## Claims

1. A two-channel sampling needle (1) for withdrawing body fluids, such as blood or tissue fluid, comprising two essentially parallel flexible cannulas (3, 12), the first cannula (3) being provided with an insertion needle (2) which may be removed after the sampling needle (1) has been introduced, the sharpened end (4) of the said insertion needle (2) projecting beyond the end of the first cannula (3), and a mounting part (8) of the sampling needle (1) being provided with fittings (10, 11) for the two cannulas (3, 12) and with a separate opening for removing the insertion needle (2), which opening is plugged by a flexible stopper (18), **wherein** the second cannula (12) is provided with at least one connecting opening (22') into the first cannula (3) at the distal end of the sampling needle (1), and wherein the second cannula (12) is located between the insertion needle (2) and the wall of the fist cannula (3).

2. A sampling needle according to claim 1, **wherein** the mounting part (8) contains a cavity (7) in connection with the first cannula (3), which cavity (7) is plugged by the flexible stopper (18) pierced by the insertion needle (2), and wherein the mounting part (8) has a projection (16) with the fittings (10, 11) for the two cannulas (3, 12), one of the said fittings (11) opening into the first cannula (3) and into the cavity (7) in the mounting part (8), and the other fitting (10) connecting to the second cannula (12).

3. A sampling needle according to claim 2, **wherein** the cavity (7) in the mounting part (8) is adjusted to the interior diameter of the first cannula (3) by means of a stepless cone (13).

4. A sampling needle according to any of claims 1 to 3, **wherein** the lumen of the second cannula (12) extends into that of the first cannula (3) only after the insertion needle (2) has been removed.

5. A sampling needle according to any of claims 2 to 4, **wherein** the projection (16) is provided with a bore (17) opening into the cavity (7), into which bore (17) a connecting piece (9) can be inserted so as to be gas-tight, holding the fittings (10, 11) or lines leading to the two cannulas (3, 12).

6. A sampling needle according to claim 5, **wherein** the connecting piece (9) with the two fittings (10, 11) is fastened to the projection (16) on the mounting part by means of a threaded part (14).

7. A sampling needle according to any of claims 1 to 6, wherein the wall of the first cannula has perforations (5).

8. A sampling needle according to any of claims 1 to 6, **wherein** the wall of the first cannula (3) has a perforation (5) covered by a membrane (19) that is permeable to selected components of the tissue fluid.

9. A sampling needle according to any of claims 1 to 8, **wherein** both fittings (10, 11) of the sampling needle (1) can be inserted into a coupling element (21) with ingoing and outgoing lines, without any risk of confusion.

## Revendications

1. Aiguille de prélèvement à double lumière pour retirer les liquides du corps par exemple du sang ou du liquide tissulaire, avec une première et une deuxième canule élastique guidées essentiellement parallèlement (3, 12), la première canule (3) présente une aiguille d'introduction (2) éliminable après l'insertion de l'aiguille de prélèvement (1) et dont l'extrémité polie (4) dépasse de la première canule, tandis qu'une pièce de prise (8) de l'aiguille de prélèvement (1) présente en plus des raccords (10, 11) des deux canules (3, 12) un orifice séparé obturé par un bouchon élastique (18) pour sortir l'aiguille d'introduction, caractérisée en ce que la deuxième canule (12) présente dans le domaine distal terminal de l'aiguille de prélèvement (1) au moins un orifice de connexion directe (22') à la première canule (3) et que la deuxième canule (12) se trouve entre l'aiguille d'introduction (2) et la paroi de la première canule (3).

2. Aiguille de prélèvement selon la revendication 1, caractérisée en ce que la pièce de prise (8) présente une chambre creuse (7) qui est en communication avec la première canule (3), et qui est obturée par le bouchon (18) élastique traversé par l'aiguille d'introduction (2), en ce que la pièce de prise (8) présente une partie d'adaptation (16) avec les raccords (10, 11) pour les deux canules (3, 12), l'une des connexions (11) débouchant dans la première canule (3) et dans la chambre creuse (7) de la pièce de prise (8) et l'autre connexion (10) étant reliée à la deuxième canule (12).

3. Aiguille de prélèvement selon la revendication 2, caractérisée en ce que la chambre creuse (7) de la pièce de prise (8) est adaptée par un cône continu (13) au diamètre interne de la première canule (3).

4. Aiguille de prélèvement selon les revendications 1 à 3 caractérisée en ce que la lumière de la deuxième canule (12) ne se développe qu'après extraction de l'aiguille d'introduction (2) dans la lumière de la première canule (3).

5. Aiguille de prélèvement selon l'une des revendications 2 à 4, caractérisée en ce que l'adaptation (16) présente un alésage (17) qui débouche dans la chambre creuse (7), tandis qu'une pièce de connexion (9) étant insérable de façon étanche aux gaz dans l'alésage (17), pièce qui présente les connexions (10, 11) ou des conduites conduisant aux deux canules (3, 12).

6. Aiguille de prélèvement selon la revendication 5, caractérisée en ce que la pièce de connexion (9) avec les deux connexions (10, 11) est fixée par une pièce filetée (14) à l'adaptation (16) de la pièce de prise.

7. Aiguille de prélèvement selon l'une des revendications 1 à 6, caractérisée en ce que la paroi de la première canule présente des orifices de paroi (5).

8. Aiguille de prélèvement selon l'une des revendications 1 à 6, caractérisée en ce que la première canule (3) présente un orifice de paroi (5) avec une membrane (19) sélectivement perméable pour certains composants du liquide tissulaire.

9. Aiguille de prélèvement selon l'une des revendications 1 à 8, caractérisée en ce qu'on peut insérer sans risque d'inversion les deux connexions (10, 11) de l'aiguille de prélèvement (1) dans une pièce d'accouplement (21) avec des conduites d'arrivée et de départ.
